# EUROPÄISCHE PATENTSCHRIFT

(11) **EP 3 154 431 B1**
(45) Veröffentlichungstag und Bekanntmachung des Hinweises auf die Patenterteilung: **11.01.2023**
(21) Anmeldenummer: 15741481.4
(22) Anmeldetag: 11.06.2015
(51) Int. Cl.: A61B 6/00, A61B 8/00, A61B 8/08, G01T 1/161, G01T 1/16

(54) **VERFAHREN ZUR BESTIMMUNG VON URSPRUNGSORTEN VON STRAHLUNGSSIGNALEN IN EINEM MESSBEREICH UND MESSGERÄT ZUR SIMULTANEN ERFASSUNG VON STRAHLUNGSEREIGNISSEN ZERFALLENDER RADIONUKLIDE IN DEM MESSBEREICH**
METHOD FOR DETERMINING THE POINTS OF ORIGIN OF RADIATION SIGNALS IN A MEASUREMENT ZONE, AND MEASURING APPARATUS FOR SIMULTANEOUSLY DETECTING RADIATION EVENTS OF DECAYING RADIONUCLIDES IN THE MEASUREMENT ZONE
PROCÉDÉ DE DÉTERMINATION DES LIEUX D'ORIGINE DE SIGNAUX DE RAYONNEMENT DANS UNE ZONE À MESURER ET APPAREIL DE MESURE PERMETTANT DE DÉTECTER SIMULTANÉMENT DES ÉVÉNEMENTS RADIATIFS DE RADIONUCLÉIDES QUI SE DÉSINTÈGRENT DANS LA ZONE À MESURER

(30) Priorität: 11.06.2014 DE 102014108178
(43) Veröffentlichungstag der Anmeldung: 19.04.2017
(73) Patentinhaber: Universitätsklinikum Jena, 07747 Jena (DE)
(72) Erfinder: FREESMEYER, Martin, 07749 Jena (DE)
(74) Vertreter: Schaller, Renate
(86) Internationale Anmeldenummer: PCT/DE2015/100233
(87) Internationale Veröffentlichungsnummer: WO 2015/188810

(56) Entgegenhaltungen:
- US-A1- 2006 237 652
- US-A1- 2009 123 048
- US-A1- 2013 172 739
- V.Y. PANIN ET AL: "Fully 3-D PET reconstruction with system matrix derived from point source measurements", IEEE TRANSACTIONS ON MEDICAL IMAGING., Bd. 25, Nr. 7, 2. Juli 2006 (2006-07-02), Seiten 907-921, XP055222064, US ISSN: 0278-0062, DOI: 10.1109/TMI.2006.876171

## Beschreibung

Verfahren zur Bestimmung von Ursprungsorten von Strahlungssignalen in einem Messbereich und Messgerät zur simultanen Erfassung von Strahlungsereignissen zerfallender Radionuklide in dem Messbereich

Die Erfindung betrifft das Gebiet der multimodalen Bildgebung, auch als Hybridbildgebung bezeichnet. Sie betrifft insbesondere ein Verfahren und eine Vorrichtung zur Bestimmung der Ursprungsorte von Strahlungssignalen in einem Messbereich, wie dies gattungsgemäß aus der US 2013 / 0 172 739 A1 bekannt ist.

Für eine Reihe von Anwendungen, beispielsweise in der Werkstoff- und Produktprüfung und in der Medizin, werden Verfahren angewendet, bei denen mittels Ultraschallsignalen in einem Messbereich Strukturen abgebildet werden, die in einer Tiefe (z-Richtung) unter der Oberfläche eines untersuchten Objektes lokalisiert sind. Ein klassisches Beispiel hierfür ist die Sonographie. Diese ist dem Wesen nach ein Verfahren der Schnittbildgebung (Ultraschalltomographie). Ein Schnittbild der Sonographie mit herkömmlichen Ultraschallsonden stellt ein Schnittbild in einem Untersuchungsobjekt, beispielsweise in einem Werkstoff oder in einem Gewebe eines Körpers, dar. Dabei werden Unterschiede der akustischen (Reflexions-)Eigenschaften im Inneren des Untersuchungsobjektes (Ultraschallsignale) visualisiert. Bei dem so erzeugten zweidimensionalen Schnittbild verläuft eine erste Dimension entlang eines Kristall-Arrays des Ultraschallkopfs (x-Richtung). Die zweite Dimension spiegelt die Verteilung der akustischen Eigenschaften in der Tiefe (z-Richtung) wider. Der so untersuchte Messbereich erstreckt sich daher vornehmlich in einer x-z-Ebene und weist nur eine geringe Dicke in y-Richtung auf.

Dagegen werden bei Verfahren auf der Basis der Detektion von Strahlungssignalen von Radionukliden flächige Abbildungen erhalten, durch die eine zweidimensionale Verteilung der Strahlungssignale und deren Intensität in einer x-y-Ebene abgebildet wird. Eine direkte Zuordnung der zweidimensionalen Verteilung zu einer Tiefenlokalisation von Ursprungsorten der Strahlungssignale in einem einzelnen Bild ist nicht möglich.

Die Informationen herkömmlicher Gammasonden werden nur als Zahl und / oder akustisches Signal (Pfiff, Klickgeräusch) ausgegeben, so dass keine Information zur Tiefenlokalisation eines Speicherherdes erhalten wird. Diese Sonden verwenden einen einzelnen Gammadetektor und einen passiven Metall-Kollimator hoher Dichte, um das Sichtfeld des Detektors zu definieren und um Untergrundstrahlung aus der Umgebung zu unterdrücken, indem diese nicht bis zu dem Detektor gelangt und von diesem nicht detektiert wird. Bei hohen Energien sind die Kollimatoren groß und schwer.

Bekannt sind Vorrichtungen, bei denen eine Ultraschallsonde und wenigstens ein Strahlungsdetektor nahe beieinander oder in einem gemeinsamen Messkopf angeordnet sind.

Eine solche Lösung ist aus der US 7 094 203 B2 bekannt. In einem Gehäuse ist einer Anzahl von Strahlungsdetektoren (Array) ein Kollimator vorgeordnet, durch dessen Wirkung ein Erfassungsbereich der Strahlungsdetektoren gerichtet und eingeschränkt wird. Unmittelbar neben dem Gehäuse ist eine Ultraschallsonde in einem gemeinsamen Messkopf angeordnet. Durch die Ultraschallsonde werden Ultraschallsignale in einem gemeinsamen Messbereich in einer x-z-Ebene erfasst. Das Array der Strahlungsdetektoren ist schwenkbar angeordnet, sodass ein Messbereich des Arrays durch den Messbereich der Ultraschallsonde gerichtet werden kann. Durch eine Schwenkbewegung des Arrays kann ein Schnittpunkt beider Messbereiche als eine Fokusebene entlang des Messbereichs der Ultraschallsonde verschoben und verschiedene Messpositionen der Strahlungsdetektoren bezüglich des gemeinsamen Messbereichs eingestellt werden. Die Informationen über die Tiefenlokalisation von Ursprungsorten können dabei durch Rekonstruktion der einzelnen Fokusebenen erhalten werden.

Ein ähnliches Verfahren ist in der EP 1 284 655 B1 beschrieben. Dabei werden eine Anzahl von Strahlungsdetektoren unter verschiedenen Winkeln in einen Messbereich gerichtet angeordnet, wobei sich die Erfassungsbereiche der Strahlungsdetektoren in dem Messbereich in einer Fokuslinie schneiden. Für die dazu zwingend benötigte Ausrichtung der Erfassungsbereiche der Strahlungsdetektoren ist jedem Strahlungsdetektor ein Kollimator vorgeordnet. Die jeweiligen Winkel der Strahlungsdetektoren sind bekannt. Unter Nutzung der Kenntnis der jeweils aktuell eingestellten Winkel kann die Lage einer aktuellen Fokuslinie ermittelt werden. Wird die Fokuslinie durch den Messbereich geführt und dabei die erfassten Strahlungssignale der jeweiligen Fokuslinie zugeordnet gespeichert, kann eine Abbildung der Radionuklidverteilung, d. h. der Ursprungsorte, rekonstruiert werden. Zusätzlich wird von dem Messbereich ein Ultraschallbild aufgenommen. Die Informationen der Ultraschalltomographie und der Rekonstruktion der Verteilung der Ursprungsorte in dem gemeinsamen Messbereich können anschließend in einem Hybridbild abgebildet werden.

Aus der vorgenannten US 2013/0 172 739 A1 ist ein Messkopf bekannt, in dem eine Ultraschallsonde neben oder in einem Array von Strahlungsdetektoren angeordnet ist. Eine Erfassung von Ultraschallsignalen und Strahlungssignalen ist nur gerichtet möglich. Die dreidimensionale Verteilung der Ursprungsorte ist nur durch eine Erfassung entsprechender Messwerte aus unterschiedlichen Messpositionen des Messkopfs und durch Rekonstruktion der an den verschiedenen Messpositionen erfassten Messwerte möglich.

Alle Lösungen des Standes der Technik erfordern eine Rekonstruktion von Daten, um eine Verteilung der Ursprungsorte und Ultraschallbilder aufgrund von Strahlungs- und Ultraschallsignalen abbilden zu können. Dabei ist eine zweifelsfreie räumliche Zuordnung nuklidmehrspeichernder Befunde zum Sonographiebild nur mit hohem Rechenaufwand und mit der sehr hohen Gefahr und der Unvorhersehbarkeit von durch die Berechnungsalgorithmen bedingten räumlichen Versetzungen möglich. Wünschenswert wäre daher eine integrierte Sonde zur möglichst gleichzeitigen Erzeugung von anatomisch korrekt übereinstimmenden Schnittbildern (gleiche Abbildungsebene) aus Gewebeechogenität (Ultraschallsignale) und Radionuklidverteilung. Dies ist derzeit im Stand der Technik nicht zufriedenstellend gelöst.

Der Erfindung liegt die Aufgabe zugrunde, eine Möglichkeit zur Erfassung von Strahlungsereignissen zerfallender Radionuklide in einem Messbereich vorzuschlagen, durch die eine vereinfachte Handhabung und eine verbesserte räumliche Auflösung von Strahlungssignalen erreicht wird.

Die Aufgabe wird durch die Gegenstände der unabhängigen Ansprüche gelöst. Vorteilhafte Ausgestaltungen sind in den abhängigen Ansprüchen angegeben.

Die Aufgabe wird in einem Verfahren gemäss Anspruch 1 gelöst.

Spezifische Daten sind vorzugsweise Daten, die einem Ursprungsort zugeordnet (ortsspezifisch) sind und die gewonnen werden, indem ein dreidimensionaler Messbereich definiert wird, in dem jeder Raumpunkt durch Koordinaten eines geeigneten Koordinatensystems, beispielsweise eines kartesischen Koordinatensystems oder eines Polarkoordinatensystems, eineindeutig beschrieben werden kann. Ein jeder Raumpunkt ist ein Ort. Zerfällt an einem solchen Raumpunkt ein Radionuklid und geht von diesem Raumpunkt ein Strahlungssignal aus, so ist dieser Raumpunkt ein Ursprungsort im Sinne dieser Beschreibung. Das erfasste Strahlungssignal verursacht in jedem Strahlungsdetektor einen Signalwert, insbesondere einen Signalwert der Intensität des erfassten Strahlungssignals. Durch jeden Strahlungsdetektor wird ein individueller Signalwert als ein Messwert erfasst und gespeichert. Die individuellen Messwerte werden zueinander in Beziehung gesetzt, die Teilreferenzdatensätze gebildet und jeweils einem der Ursprungsorte zugeordnet gespeichert. Zusätzlich können die Signalwerte in den Teilreferenzdatensätzen abgespeichert werden, um Daten der Intensitäten der erfassten Strahlungssignale verfügbar zu haben und abbilden zu können. Vorzugsweise werden die Intensitäten durch geeignete farbige und / oder sonstige grafische Hervorhebungen, wie Schattierungen, Umrahmungen, Grauwertdarstellungen, blinkende Darstellungen usw., in einer Abbildung der Ursprungsorte betont.

Die vorstehenden Überlegungen gehen von einem idealisierten Zustand aus. Da tatsächlich ein Radionuklid eine mehr als punktförmige Größe besitzt und sich eine Quelle eines Strahlungssignals messtechnisch kaum auf einen Raumpunkt reduzieren lässt, wird in dieser Beschreibung unter einem Ursprungsort auch ein dreidimensionaler Bereich verstanden, aus dem das Strahlungssignal hervorgeht.

Ein Messbereich ist ein Ausschnitt einer sich in einer x-z-Ebene eines kartesischen Koordinatensystems erstreckenden Schicht. Die Dicke der Schicht in y-Richtung beträgt beispielsweise 1 mm, 3 mm oder 5 mm.

Mittels des erfindungsgemäßen Verfahrens sind vorteilhaft eine Erfassung und Bestimmung einer zweidimensionalen Intensitätsverteilung der erfassten Strahlungssignale des Radionuklids im gesamten Messbereich ermöglicht. Aufgrund der erfassten Strahlungssignale und deren Intensitäten ist daher auch die zweidimensionale Verteilung des Radionuklids in dem Messbereich ableitbar. Für jeden Raumpunkt in dem Messbereich kann die Intensität der Strahlungssignale (Radioaktivitätsgehalt) bestimmt und je einer zweidimensionalen Position in dem Messbereich zugeordnet werden. Positionen und Intensitäten können in einer Bildmatrix dargestellt werden. Dabei werden unterschiedliche Intensitäten vorzugsweise farb- oder graustufencodiert abgebildet.

Mittels des erfindungsgemäßen Verfahrens ist es auch möglich, mehrere nebeneinander liegende Messbereiche zu definieren und anhand der bereitgestellten Messwerte eine dreidimensionale Verteilung der Ursprungsorte sowie deren jeweiliger Intensität zu erstellen.

Da individuelle technische Bauelemente wie Strahlungsdetektoren hinsichtlich ihrer Kennkurven nicht absolut identisch zueinander sind, ist es vorteilhaft, wenn die je Strahlungsdetektor bereitgestellten Messwerte bei Bedarf kalibriert werden. So können auftretende Unterschiede in der individuellen Effizienz der Erfassung von Strahlungssignalen der Strahlungsdetektoren kalibriert werden, indem jeder Messwert mit einem Kalibrierungsfaktor verrechnet, beispielsweise multipliziert wird. Die kalibrierten Messwerte werden für die Ermittlung des Referenzdatensatzes verwendet.

Außerdem kann es vorkommen, dass durch die Strahlungsdetektoren Signalwerte erfasst und Messwerte bereitgestellt werden, obwohl aus dem Messbereich gar keine Strahlungssignale ausgesendet werden. Dieser Effekt kann beispielsweise auf einer Erfassung von Strahlungssignalen von außerhalb des Messbereichs sowie auf physikalischen und chemischen Vorgängen innerhalb der Strahlungsdetektoren beruhen. Solche als Untergrund-Messwerte bezeichneten Daten werden vorteilhaft ebenfalls ermittelt und bei der Erstellung des Referenzdatensatzes berücksichtigt, indem die Messwerte bezüglich der Untergrund-Messwerte korrigiert werden.

Ferner ist es möglich, dass sowohl die Kalibrierungsfaktoren als auch die Untergrund-Messwerte über die Dauer der Verfahrensdurchführung und / oder über die Lebensdauer eines Strahlungsdetektors (sogenannte Langzeitdrift) angepasst werden bzw. angepasst werden müssen.

Die vorgenannten individuellen Anpassungen und Korrekturen können auch bei der Bereitstellung der Messwerte vorgenommen und / oder berücksichtigt werden.

Als durch die Strahlungsdetektoren bereitgestellte Messwerte werden auch Messwerte angesehen, die signaltechnisch aufbereitet wurden. So können erfasste Strahlungssignale als Signalwerte erfasst und anschließend gefiltert, transformiert und / oder geglättet werden, bevor sie als Messwerte bereitgestellt werden.

Es ist ferner sehr vorteilhaft, wenn nicht aufgrund eines einzelnen erfassten Strahlungssignals und des daraus resultierenden Signalwertes ein Messwert bereitgestellt wird, sondern ein Messwert beispielsweise als ein Mittelwert einer Anzahl von erfassten Strahlungssignalen bereitgestellt wird. Als Mittelwerte können beispielsweise arithmetische, gewichtete, gleitende oder geometrische Mittelwerte sowie Abwandlungen dieser verwendet werden. Da die Bildung von Mittelwerten bedingt, dass eine Anzahl von Messwerten von verschiedenen Messzeitpunkten vorliegt, ist unter dem Begriff des Messzeitpunkts neben einem einzelnen Zeitpunkt, zu dem eine Messung erfolgt, auch ein Zeitraum zu verstehen, über den eine Anzahl von Messungen erfolgen.

Ein Referenzdatensatz wird beispielsweise dadurch bereitgestellt, dass in einem Messbereich an einem bekannten Ursprungsort eine bekannte Menge eines oder mehrerer Radionuklide platziert wird und die aufgrund der erfassten Strahlungssignale je Strahlungsdetektor bereitgestellten Messwerte dem Ursprungsort und einem Messzeitpunkt zugeordnet abgespeichert werden. Dabei sind die Strahlungsdetektoren in einer bekannten relativen Lage zu dem Messbereich angeordnet und zu diesem ausgerichtet (Messposition). Anschließend werden die bereitgestellten Messwerte der Strahlungsdetektoren nach vorbestimmten Regeln in Beziehung zueinander gesetzt. Solche Beziehungen sind mathematische Beziehungen, wie beispielsweise Quotienten, Differenzen, Summen, Produkte sowie Kombinationen daraus. Diese Beziehungen und deren jeweilige Ergebnisse werden als Teilreferenzdatensätze dem jeweiligen Ursprungsort zugeordnet gespeichert. Den Teilreferenzdatensätzen können zusätzliche Daten, wie zum Beispiel die Art und die Menge der eingesetzten Radionuklide, sowie weitere Daten zu den Bedingungen während der Erstellung des Referenzdatensatzes zugeordnet und wiederholt abrufbar gespeichert werden.

Anschließend wird das vorstehend beschriebene Vorgehen für weitere Ursprungsorte wiederholt, bis ein Referenzdatensatz mit einer gewünschten räumlichen Auflösung der Ursprungsorte erstellt ist.

Bei der Erstellung des Referenzdatensatzes können zudem Kalibrierungsfaktoren und Untergrund-Messwerte ermittelt werden. Langzeitdriften können durch mehrmaliges, zeitlich versetztes Erstellen von Referenzdatensätzen ermittelt oder abgeschätzt werden. Dabei können Referenzdatensätze, die zu unterschiedlichen Zeitpunkten erstellt wurden, miteinander verglichen und festgestellte systematische Abweichungen der Messwerte zur Erkennung und Korrektur von Langzeitdriften verwendet werden.

Es ist auch möglich, dass für einige oder alle Ursprungsorte eine rechnergestützte Simulation durchgeführt wird und die bereitgestellten Messwerte anhand der Simulation ermittelt werden. Ferner ist es möglich, dass für eine bestimmte Auswahl von Ursprungsorten tatsächlich Strahlungssignale erfasst und beispielsweise Kalibrierungsfaktoren und Untergrund-Messwerte ermittelt werden. Anhand dieser ausgewählten Ursprungsorte können Kalibrierungsfaktoren und / oder Untergrund-Messwerte für den Referenzdatensatz extrapoliert werden.

Durch das erfindungsgemäße Verfahren ist es vorteilhaft ermöglicht, Ursprungsorte von Strahlungssignalen zu ermitteln, ohne dabei eine Messposition der Strahlungsdetektoren verändern zu müssen.

Die zu einem Messzeitpunkt bestimmten Ursprungsorte der erfassten Strahlungssignale können in weiteren Ausgestaltungen des erfindungsgemäßen Verfahrens für eine graphische Darstellung des Messbereichs mit den Ursprungsorten als Abbildungsdaten bereitgestellt werden. Graphische Darstellungen können beispielsweise auf einem Monitor, als Projektion und / oder als gedruckte Wiedergabe von Messbereich und Ursprungsorten erfolgen.

Es ist in einer vorteilhaften Weiterbildung des erfindungsgemäßen Verfahrens möglich, dass zu dem Messzeitpunkt zusätzlich Ultraschallsignale aus dem Messbereich erfasst und als dem Messzeitpunkt zugeordnete Ultraschallmesswerte für eine graphische Darstellung des Messbereichs bereitgestellt werden. Dabei werden die Ursprungsorte der Strahlungssignale und die Ultraschallmesswerte zeitgleich, orts- und lagerichtig in einer graphischen Darstellung angezeigt. Vorzugsweise werden diese Daten in einem Hybridbild abgebildet.

Es wird mittels der vorstehend angegebenen Weiterbildung des erfindungsgemäßen Verfahrens also ermöglicht, eine Abbildung der Intensitätsverteilung der erfassten Strahlungssignale (Bildmatrix) in dem gemeinsamen Messbereich, hier also in der x-z-Ebene des Ultraschalls, zu erzeugen.

Ein Messbereich ist vorzugsweise durch einen Ausschnitt eines Erfassungsbereiches der Strahlungsdetektoren gegeben, in dem eine effektive und genaue Erfassung und Lokalisation der Ursprungsorte möglich ist. Durch die Gestaltung des erfindungsgemäßen Verfahrens ist vorteilhaft eine Detektion der Ursprungsorte auch in der z-Richtung ermöglicht. Da eine Erfassung von Ultraschallsignalen ebenfalls in der z-Richtung erfolgt, ist es möglich, simultan in einem Messbereich, der eine Ausdehnung vornehmlich in einer x-z-Ebene aufweist, sowohl Ursprungsorte von Strahlungssignalen als auch Ultraschallsignale zu bestimmen bzw. zu erfassen und (gemeinsam) abzubilden.

Durch eine solche Ausgestaltung des erfindungsgemäßen Verfahrens ist eine sehr vorteilhafte Kombination zweier Messverfahren sowie die simultane Darstellung der Ursprungsorte und der Ultraschallmesswerte erreicht. Bei Bedarf können zudem die Messwerte der Strahlungssignale wie Intensitäten der Signalwerte in geeigneter Weise dargestellt werden. So können diese als Zahlenwerte sowie in Form eines Farbkodes, beispielsweise farbig kodiert oder als Grauwerte abgebildet werden.

Im Gegensatz zu bekannten Verfahren des Standes der Technik ist es bei dem erfindungsgemäßen Verfahren nicht erforderlich, die Strahlungsmesswerte und die Ultraschallmesswerte von unterschiedlichen Messpositionen aus zu erfassen, um eine Abbildung generieren zu können. Abbildungen sind in Echtzeit möglich, wobei Abbildungen mit leichten rechentechnisch bedingten zeitlichen Verzögerungen trotzdem als in Echtzeit bereitgestellt gelten. Sehr günstig ist zudem, dass durch das erfindungsgemäße Verfahren keine Rekonstruktion der Abbildungen erforderlich ist. Dadurch ist ein fehlerhafter räumlicher Versatz der abgebildeten Strahlungsmesswerte und gegebenenfalls der Ultraschallmesswerte zueinander, der aufgrund von Vereinfachungen in den für die Rekonstruktion verwendeten Berechnungsalgorithmen häufig auftritt, vorteilhaft reduziert oder gänzlich vermieden.

Es ist ferner möglich, dass die Strahlungsdetektoren ohne einen den Strahlungsdetektoren empfangsseitig vorgeordneten Kollimator angeordnet werden. Durch eine solche Ausgestaltung des Verfahrens ist die Verwendung einer leichteren Vorrichtung möglich und eine Einschränkung des Empfangsbereichs der Strahlungsdetektoren vermieden.

Das erfindungsgemäße Verfahren kann zur Ermittlung von Ursprungsorten von Strahlungssignalen und damit zur Ermittlung der räumlichen Verteilung von Radionukliden in einem Messbereich genutzt werden. Anwendungen können beispielsweise auf dem Gebiet der Messtechnik, der Material- und Qualitätskontrolle sowie der Medizin liegen.

Insbesondere bei einer Kombination des erfindungsgemäßen Verfahrens mit der zeitgleichen Erfassung und Abbildung von Ultraschallmesswerten eröffnet sich für medizinische Anwendungen vorteilhaft die Möglichkeit, Strahlungsmesswerte und Ultraschallmesswerte simultan erfassen und abbilden zu können, ohne dabei Daten von einer Mehrzahl von Messpositionen aus erfassen zu müssen.

Die Aufgabe wird zudem mit einem Messgerät gemäss Anspruch 5 gelöst.

Die Anzahl n der Strahlungsdetektoren beträgt wenigstens zwei, was nachfolgend durch die Angabe n = 2, 3, ..., i ausgedrückt ist.

Die Strahlungsdetektoren sind vorteilhaft Detektoren, mit denen eine Intensität der erfassten Strahlungssignale als Messwert bereitgestellt werden kann. Solche Detektoren sind beispielsweise Hableiterdetektoren wie CdZnTe-Detektoren oder HgJ₂-Detektoren. Bei diesen wird durch eine auftreffende Strahlung (Strahlungssignal), beispielsweise Gammastrahlung, auf dem Halbleitermaterial ein Photoeffekt ausgelöst, dessen Stärke proportional zur Intensität des Strahlungssignals ist. Eine Intensitätsmessung erfolgt durch Messung eines durch den Photoeffekt ausgelösten Stromflusses (Stromimpuls).

Ebenfalls geeignete Detektoren sind Szintillationsdetektoren, beispielsweise Bi₄Ge₃O₁₂-, Lu₂SiO₅:Ce-, Lu_{0,8}Y_{0,2}AlO₃:Ce-, Gd₂SiO₅:Ce-, CsJ:TI- oder NaJ:TI-Detektoren. Diese Materialien weisen eine hohe Lichtausbeute und geringe Abklingzeiten auf. In Szintillationsdetektoren wird eine durch ein erfasstes Strahlungssignal verursachte Ionisation in Lichtblitze umgesetzt, die durch eine geeignete Photodiode oder einen Photomultiplier detektiert werden. Die Helligkeit der detektierten Lichtblitze ist proportional zur Intensität des Strahlungssignals.

In einer günstigen Ausführung des erfindungsgemäßen Messgeräts ist die Auswerteeinheit so konfiguriert, dass die Bestimmung des Ursprungsortes eines jeden Strahlungssignals unter Nutzung des Referenzdatensatzes erfolgt, wobei die spezifischen Daten jedes Teilreferenzdatensatzes durch Beziehungen von Messwerten (siehe oben) der Strahlungsdetektoren zueinander gebildet sind.

In dem Messkopf kann in einer weiteren Ausführung zusätzlich eine Ultraschallsonde zum Senden und Empfangen von Ultraschallsignalen aus dem Messbereich angeordnet sein. Als Messkopf kann ein bekannter Ultraschallkopf verwendet werden, in den ein Array von Strahlungsdetektoren integriert ist. Ein Array ist eine Anordnung mindestens zweier Strahlungsdetektoren, die in einer festen Lage zueinander angeordnet sind.

Eine einfachere, weil leichte und mit weniger Bauteilen realisierte Ausführung des erfindungsgemäßen Messgeräts besteht darin, dass das Messgerät als ein kollimatorloses Messgerät ausgebildet ist. Dies ist vorteilhaft durch die Anzahl der Strahlungsdetektoren und durch die Konfiguration der Auswerteeinheit erreicht. Eine Kollimation der aus dem Messbereich den Messkopf erreichenden Signale, insbesondere der Strahlungssignale, kann unterbleiben, da die erfindungsgemäße Konfiguration der Auswerteeinheit im Zusammenwirken mit der bekannten relativen räumlichen Anordnung der Strahlungsdetektoren zueinander eine hinreichend präzise Ermittlung der Ursprungsorte erlaubt.

So liegt die Präzision der Ermittlung der Ursprungsorte in vorteilhaften Ausführungen der erfindungsgemäßen Vorrichtung bei weniger als 5 mm bis 10 mm.

In einer sehr vorteilhaften, weil für die praktische Anwendung bedeutsamen Ausführung des Messgeräts ist der Messkopf ein tragbares Gerät, das durch eine Hand eines Benutzers haltbar und führbar ist. In einer bevorzugten Ausführung entspricht das Messgerät in Form und Dimension einer Ultraschallsonde, wie diese aus dem Stand der Technik bekannt ist und wie diese insbesondere im Bereich der Medizin für Ultraschalluntersuchungen an Weichgeweben verwendet wird.

Die Strahlungsdetektoren können dabei in unterschiedlichen Anordnungen vorhanden sein. In einer Ausführung des erfindungsgemäßen Messgeräts sind die Strahlungsdetektoren in mindestens einem Array angeordnet.

In weiteren Ausführungen können die Strahlungsdetektoren um die mindestens eine vorhandene Ultraschallsonde angeordnet sein, sodass diese in einem Array von Strahlungsdetektoren angeordnet ist.

Die mindestens eine Ultraschallsonde kann in einer weiteren Ausführung auch neben einem Array von Strahlungsdetektoren angeordnet sein. Darüber hinaus ist es möglich, dass das Array von Strahlungsdetektoren hinter der mindestens einen Ultraschallsonde angeordnet ist, sodass die Strahlungssignale durch Bereiche der Ultraschallsonde hindurchtreten, bevor sie durch die Strahlungsdetektoren erfassbar sind. Diese Ausführung erlaubt eine besonders kompakte Bauform.

Die Erfindung wird nachstehend anhand von Ausführungsbeispielen und Abbildungen näher beschrieben. Dabei zeigen:
- Fig. 1: ein Ausführungsbeispiel eines erfindungsgemäßen Messgeräts mit einem Array von Strahlungsdetektoren in einem Messkopf,
- Fig. 2: eine schematische Darstellung von Detektionskurven von fünf Strahlungsdetektoren,
- Fig. 3: ein erstes Ausführungsbeispiel eines erfindungsgemäßen Messgeräts mit einem Array von Strahlungsdetektoren und einer vor dem Array angeordneten Ultraschallsonde in einem Messkopf,
- Fig. 4: ein zweites Ausführungsbeispiel eines erfindungsgemäßen Messgeräts mit einem Array von Strahlungsdetektoren und einer neben dem Array angeordneten Ultraschallsonde in einem Messkopf und
- Fig. 5: ein drittes Ausführungsbeispiel eines erfindungsgemäßen Messgeräts mit einem Array von Strahlungsdetektoren und einer in dem Array angeordneten Ultraschallsonde in einem Messkopf.

Die Ausführungsbeispiele sind schematisch und nicht maßstabsgetreu dargestellt. Dabei bezeichnen gleiche Bezugszeichen immer gleiche technische Merkmale, falls dies nicht ausdrücklich anders angegeben ist.

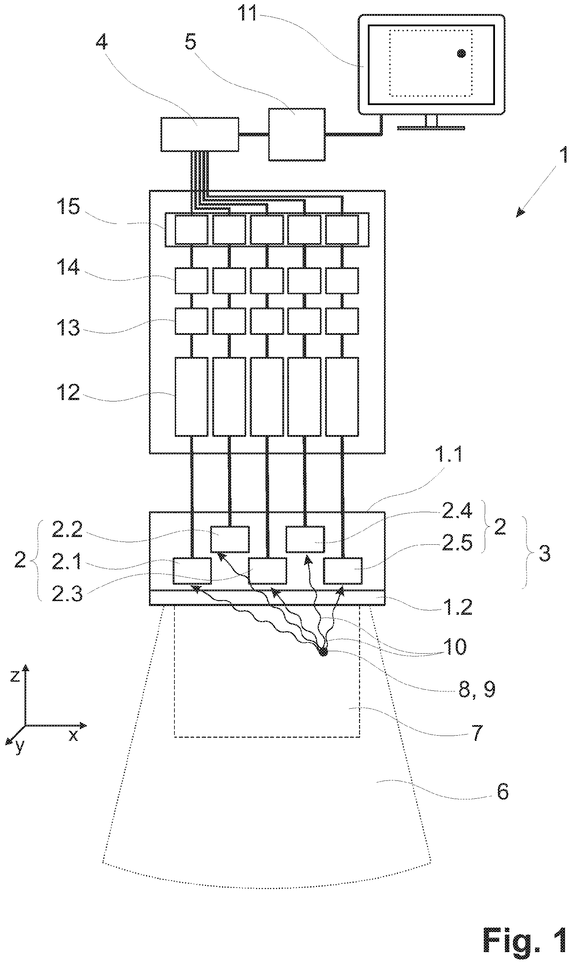

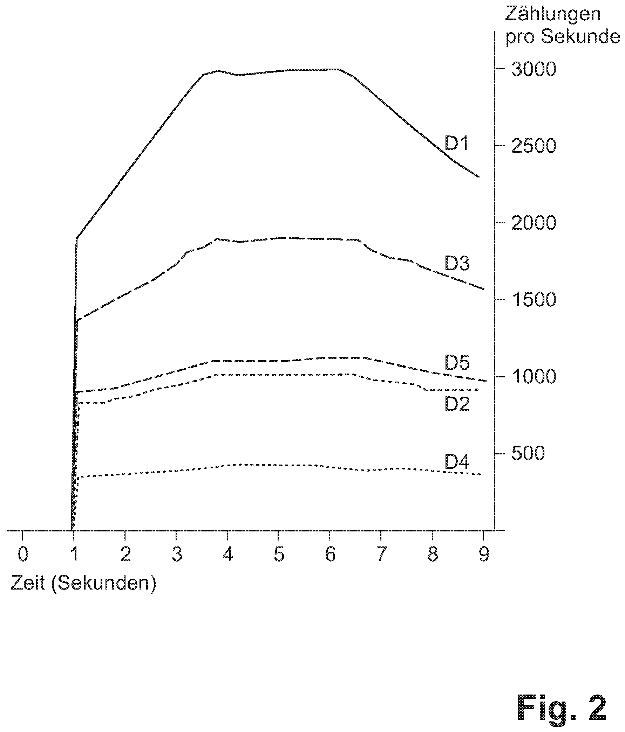

Ein Ausführungsbeispiel eines erfindungsgemäßen Messgeräts 1 wird anhand der Figuren 1 und 2 erläutert. Das Messgerät 1, das als ein manuell zu führendes Echtzeit-Emissionsorterfassungsgerät (real time handheld Emission Spot Allocator, rthESA) ausgeführt ist, besteht aus drei Hauptkomponenten: einem Array 3 von Strahlungsdetektoren 2, einer elektronischen Auswerteeinheit 4 und einer rechnerunterstützten Arbeitsstation 5.

Das Array 3 ist in einem Gehäuse eines Messkopfes 1.1 angeordnet und besteht im Ausführungsbeispiel aus einem ersten Strahlungsdetektor 2.1, einem zweiten Strahlungsdetektor 2.2, einem dritten Strahlungsdetektor 2.3, einem vierten Strahlungsdetektor 2.4 und einem fünften Strahlungsdetektor 2.5. Die ersten bis fünften Strahlungsdetektoren 2.1 bis 2.5 sind CdZnTe-Halbleiterdetektoren mit einer Kantenlänge von 4,8 mm, die in einer senkrecht auf einem Eintrittsfenster 1.2 des Messkopfes 1.1 stehenden x-z-Ebene alternierend angeordnet sind. Dabei sind der erste, der dritte und der fünfte Strahlungsdetektor 2.1, 2.3 und 2.5 direkt an dem Eintrittsfenster 1.2 des Messkopfes 1.1 angeordnet (vordere Detektoren), während der zweite und der vierte Strahlungsdetektor 2.2. und 2.4 weiter von dem Eintrittsfenster 1.2 entfernt angeordnet sind (hintere Detektoren). Der erste und der dritte Strahlungsdetektor 2.1, 2.3 sowie der dritte und der fünfte Strahlungsdetektor 2.3, 2.5 sind zueinander mit je einer Lücke angeordnet, über der der zweite Strahlungsdetektor 2.2 bzw. der vierte Strahlungsdetektor 2.4 angeordnet ist. Die x-z-Ebene, in der die ersten bis fünften Strahlungsdetektoren 2.1 bis 2.5 des Arrays 3 angeordnet sind, erstreckt sich in Richtung der x-Achse und in Richtung der z-Achse eines dem Messgerät 1 zugeordneten kartesischen Koordinatensystems.

Je nachdem, von welchem der Ursprungsorte 9 ein Strahlungssignal 10 (durch geschweifte Pfeile symbolisiert) eines Radionuklids 8 (vereinfachend zusammen mit dem Ursprungsort 9 dargestellt) durch die ersten bis fünften Strahlungsdetektoren 2.1 bis 2.5 erfasst wird, musste durch das betreffende Strahlungssignal 10 eine unterschiedlich lange Wegstrecke bis zu den jeweiligen ersten bis fünften Strahlungsdetektoren 2.1 bis 2.5 zurückgelegt werden. Dabei unterschieden sich auch die Anteile der Wegstrecken, die innerhalb eines Mediums des Messbereichs 7 zurückzulegen waren. Außerdem trifft ein Strahlungssignal 10 unter verschiedenen Winkeln auf die ersten bis fünften Strahlungsdetektoren 2.1 bis 2.5 auf, wodurch die Wirkung des Strahlungssignals 10 auf den jeweiligen ersten bis fünften Strahlungsdetektor 2.1 bis 2.5, also die geometrische Effizienz des betreffenden ersten bis fünften Strahlungsdetektors 2.1 bis 2.5 bezüglich der Erfassung eines Strahlungssignals 10 von einem bestimmten der Ursprungsorte 9, verschieden und von der Lokalisation dieses Ursprungsorts 9 abhängig ist.

Die CdZnTe-Halbleiterdetektoren (Reaktionszeit < 80 ns; Totzeit 20 µs) sind sehr kompakt, weisen eine hohe Effizienz (ca. 80 %) auf und benötigen keine platzraubenden Photomultiplier. Die Strahlungsdetektoren 2 sind in der Lage, 100 bis 50.000 Ereignisse je Sekunde (Zählungen pro Sekunde, counts per second, cps) zu zählen. Die totale Totzeit des Messgeräts 1 beträgt 20 µs. In weiteren Ausführungen des erfindungsgemäßen Messgeräts 1 können andere Strahlungsdetektoren 2 mit anderen technischen Parametern angeordnet sein.

Das messbare Energiespektrum reicht von 100 bis 550 keV. Die ersten bis fünften Strahlungsdetektoren 2.1 bis 2.5 wurden mit einer umgekehrten Vorspannung (reverse bias) betrieben und mit fünffachen Vorverstärkern 12 (12 V, Niedrigspannung) verbunden. Die aufgrund erfasster Strahlungssignale 10 in den jeweiligen Strahlungsdetektoren 2 bewirkten Signale der ersten bis fünften Strahlungsdetektoren 2.1 bis 2.5 werden getrennt voneinander verstärkt und weitergeleitet. Durch jeden Vorverstärker 12 werden die jeweiligen Strompulse der ersten bis fünften Strahlungsdetektoren 2.1 bis 2.5 in einen Spannungspuls umgewandelt. Da diese Spannungspulse einen hohen Rauschanteil aufweisen, sind Modulatoren 13 mit einem Bandpassfilter nachgeschaltet, durch die der jeweilige Spannungspuls in ein Signal mit einer Gaußverteilung gewandelt wird. Durch je einen nachgeschalteten Diskriminator 14 werden alle Signale unter einem Schwellwert von 40 keV ausgeschaltet und das Signal mit einer Gaußverteilung in einen logischen Puls (Puls vorhanden: Ja / Nein; I/0) gewandelt, der von einer Zählerkarte 15 gezählt und das Zählereignis einem jeweiligen Messzeitpunkt zugeordnet gespeichert wird. Die Zählerkarte 15 ist signaltechnisch mit einer Auswerteeinheit 4 und mit einer rechnerunterstützten Arbeitsstation 5 verbunden. Die Arbeitsstation 5 weist eine Anzeige 11 zur bildlichen Darstellung der Ursprungsorte 9 der Strahlungssignale 10 und deren jeweiliger Intensitäten auf.

Die Auswerteeinheit 4 ist so konfiguriert, dass durch diese für jeden der ersten bis fünften Strahlungsdetektoren 2.1 bis 2.5 die Zählereignisse je Sekunde (cps) erfasst und für jeden der ersten bis fünften Strahlungsdetektoren 2.1 bis 2.5 gleitende Mittelwerte der Zählereignisse je Sekunde berechnet und einem Messzeitpunkt oder einem Messzeitraum zugeordnet gespeichert werden. Die fünf Mittelwerte stellen die von den ersten bis fünften Strahlungsdetektoren 2.1 bis 2.5 bereitgestellten Messwerte dar. Es werden mathematische Beziehungen als Quotienten der Mittelwerte berechnet, wobei die Quotienten aus verschiedenen festgelegten Kombinationen berechnet werden. Unter diesen Kombinationen sind Quotienten der ersten, dritten und fünften Strahlungsdetektoren 2.1, 2.3 und 2.5 sowie der zweiten und vierten Strahlungsdetektoren 2.2 und 2.4.

Für die festgelegten Beziehungen wurde ein Referenzdatensatz von dreiundsechzig Ursprungsorten 9 ermittelt und für Vergleichszwecke wiederholt abrufbar gespeichert. Der Referenzdatensatz enthält je Ursprungsort 9 einen Teilreferenzdatensatz, der durch dieselben Quotienten von Kombinationen von Messwerten gebildet ist, wie dies vorstehend erläutert wurde. Anhand eines Vergleichs der zu einem Messzeitpunkt erhaltenen Beziehungen mit den Teilreferenzdatensätzen wird eine bestmögliche Übereinstimmung der Beziehungen mit den Teilreferenzdatensätzen ausgewählt und so der Ursprungsort 9 des erfassten Strahlungssignals 10 ermittelt.

Durch die Strahlungsdetektoren 2 sind grundsätzlich Strahlungssignale 10 aus einem etwa halbkreisförmigen Erfassungsbereich 6 detektierbar. Reproduzierbare Ergebnisse werden jedoch nur aus einem kleineren Bereich erhalten. Aus diesem Grunde ist ein etwa quaderförmiger Messbereich 7 mit einer Ausdehnung von rund 4 cm in Richtung der x-Achse und 3 cm in Richtung der z-Achse festgelegt. Der Messbereich 7 hat in Richtung der y-Achse eine Ausdehnung von rund einem Millimeter. Da der Messbereich 7 etwa 1 cm unterhalb der vorderen Detektoren beginnt, beträgt die gesamte Messtiefe in Richtung der z-Achse etwa 4 cm. Durch die bekannte zueinander versetzte räumliche Anordnung der ersten bis fünften Strahlungsdetektoren 2.1 bis 2.5 ist in Verbindung mit einem Vergleich der Beziehungen der Messwerte mit den Teilreferenzdatensätzen eine Lokalisation des Ursprungsortes 9 eines erfassten Strahlungssignals 10 ermittelbar.

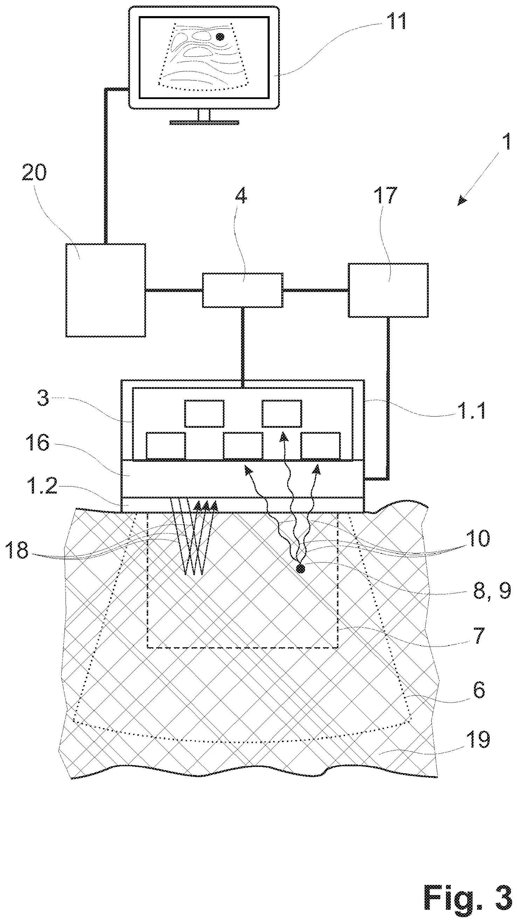

Der Referenzdatensatz mit den Teilreferenzdatensätzen wird anhand unterschiedlicher Systeme erstellt. Ein erstes System wird unter normalen Umgebungsbedingungen und Luftatmosphäre (air phantom), ein zweites System wird zur Simulation von weichem Gewebe in Wasser (water phantom) und ein drittes System wird in einem Probekörper 19 (siehe Fig. 3) etabliert. Als Probekörper 19 ist die Leber eines Tieres, beispielsweise eines Rindes, verwendet.

Das erste und das zweite System (beide nicht gezeigt) sind durch eine vertikal ausgerichtete Plexiglasplatte gebildet, die Löcher an bekannten Positionen aufweist. In eines der Löcher, das den betreffenden Ursprungsort 9 darstellt, wird eine Kapsel mit Radionukliden 8 (vereinfachend gleich dem Ursprungsort 9 angenommen) eingesteckt, die von den zerfallenden Radionukliden 8 ausgehenden Strahlungssignale 10 werden erfasst, gezählt und je erstem bis fünftem Strahlungsdetektor 2.1 bis 2.5 einem Messzeitpunkt zugeordnet gespeichert. Die Erfassung erfolgt von einer Messposition des Messkopfes 1.1. Von den gespeicherten Daten werden gleitende Mittelwerte berechnet und von diesen die festgelegten mathematischen Beziehungen gebildet. Die so ermittelten spezifischen Daten werden in einem Teilreferenzdatensatz abgelegt. Anschließend wird die Kapsel mit Radionukliden 8 in ein anderes der Löcher der Plexiglasplatte gesteckt und die vorbeschriebene Prozedur wiederholt. Zur Ermittlung eines solchen Referenzdatensatzes werden gemäß dem Ausführungsbeispiel verschiedene Radionuklide 8 (^{99m}Tc, ¹³¹I, ¹⁸F mit je 1 MBq) verwendet, wobei für jedes Radionuklid 8 ein eigener Referenzdatensatz ermittelt wird.

Das dritte System ist durch den Probekörper 19 (siehe Fig. 3), beispielweise eine Rinderleber, gebildet. In diese wird eine Kapsel mit einem Radionuklid 8 (^{99m}Tc -Lösung, 1 MBq) gesteckt. Mittels des dritten Systems ist eine Überprüfung der Übereinstimmung eines jeweiligen Ursprungsortes 9 in einem Gewebe mit der Lokalisation dieses Ursprungsortes 9 durch das erfindungsgemäße Verfahren ermöglicht.

In Fig. 2 sind schematisch Messwerte als erste bis fünfte Detektionskurven D1 bis D5 in Form gleitender Mittelwerte der Zählereignisse je Sekunde (cps) über der Zeit [Sekunden] aufgetragen. Die Zählereignisse sind daher Messzeitpunkten zugeordnet. Die erste Detektionskurve D1 zeigt die für den ersten Strahlungsdetektor 2.1 erfassten Zählereignisse, die zweite Detektionskurve D2 zeigt die für den zweiten Strahlungsdetektor 2.2 erfassten Zählereignisse, bis hin zur fünften Detektionskurve D5, durch welche die für den fünften Strahlungsdetektor 2.5 erfassten Zählereignisse dargestellt sind.

Die Verläufe der einzelnen ersten bis fünften Detektionskurven D1 bis D5 lassen erkennen, dass durch die einzelnen ersten bis fünften Strahlungsdetektoren 2.1 bis 2.5 über die Dauer der Messungen (hier etwa acht Sekunden) mehr oder weniger starke Unterschiede der Zählereignisse auftreten. Zur Ermittlung spezifischer Daten werden die Mittelwerte der Messzeitpunkte von vier bis sechs Sekunden als Messwerte genutzt, die zueinander in Beziehung gesetzt werden. Die Ergebnisse dieser Beziehungen werden als Teilreferenzdatensatz dem Ursprungsort 9 sowie dem Messzeitpunkt zugeordnet als spezifische Daten des Ursprungsortes 9 in dem Teilreferenzdatensatz abgespeichert.

Ist in einer weiteren Ausführung des erfindungsgemäßen Messgeräts 1 gemäß Fig. 3 zusätzlich zu dem Array 3 eine Ultraschallsonde 16 angeordnet und werden zeitgleich zu den Strahlungssignalen 10 auch Ultraschallsignale 18 (durch gerade Pfeile symbolisiert) erfasst, können aufgrund beider Signalarten Hybridbilder generiert und angezeigt werden. Dabei werden die Intensitäten der Strahlungssignale 10 ihren Ursprungsorten 9 zugeordnet dargestellt, indem diese entsprechend der Intensität farbig und / oder durch entsprechende Helligkeitswerte abgebildet werden. Zugleich ist die Abbildung der Ursprungsorte 9 orts- und lagerichtig von einer Abbildung der Ultraschallsignale 18 überlagert, wobei die Kontraste und Helligkeitswerte so gewählt sind, dass die Abbildungen aufgrund der Strahlungssignale 10 und der Ultraschallsignale 18 gleichzeitig visuell wahrnehmbar sind. Eine anatomisch orts- und lagerichtige Abbildung ist durch eine Bildverarbeitungseinheit 20 bewirkt, durch welche die bereitgestellten Messwerte der Strahlungssignale 10 und die Befunde aufgrund der Ultraschallsignale 18 in dem Messbereich 7 fusioniert werden.

In dem in Fig. 3 gezeigten ersten Ausführungsbeispiel eines erfindungsgemäßen Messgeräts 1 ist die Ultraschallsonde 16 in dem Messkopf 1.1 platzsparend vor dem Array 3 angeordnet.

In dem Erfassungsbereich 6 ist als ein drittes System eine Rinderleber als Probekörper 19 vorhanden. In dem Probekörper 19 ist eine Kapsel (nur angedeutet gezeigt) mit einer ^{99m}Tc-Lösung (1 MBq) an einem Ort mit bekannten Koordinaten eingesteckt. Mittels des dritten Systems kann in einem solchen Fall auch der Grad der Übereinstimmung der Abbildung der Ursprungsorte 9 der Strahlungssignale 10 sowie deren Intensität mit der Abbildung der durch die Ultraschallsignale 18 detektierten Gewebestrukturen (Befunde) überprüft werden.

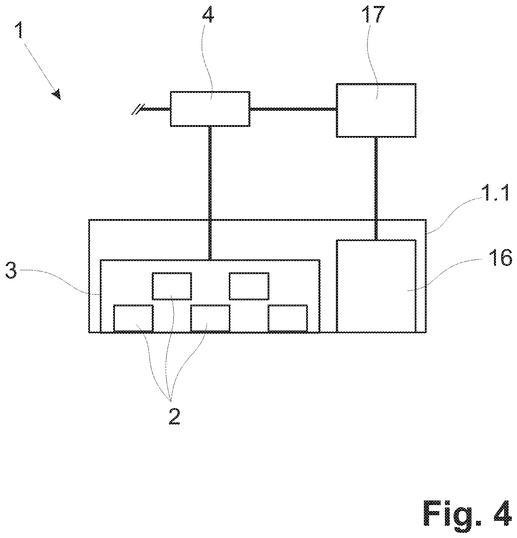

Ein zweites Ausführungsbeispiel eines erfindungsgemäßen Messgeräts 1 mit einem Array 3 von Strahlungsdetektoren 2 und einer neben dem Array 3 angeordneten Ultraschallsonde 16 in einem Messkopf 1.1 ist in Fig. 4 gezeigt. Dabei sind vereinfachend nur schematisch einige Elemente des Messgeräts 1, wie die Strahlungsdetektoren 2, das Array 3 und die Ultraschallsonde 16, dargestellt. Die Ultraschallsonde 16 ist in dem Messkopf 1.1 integriert und in bekannter Weise an ein Ultraschallgerät 17 angeschlossen. Dieses steht signaltechnisch mit der Auswerteeinheit 4 in Verbindung.

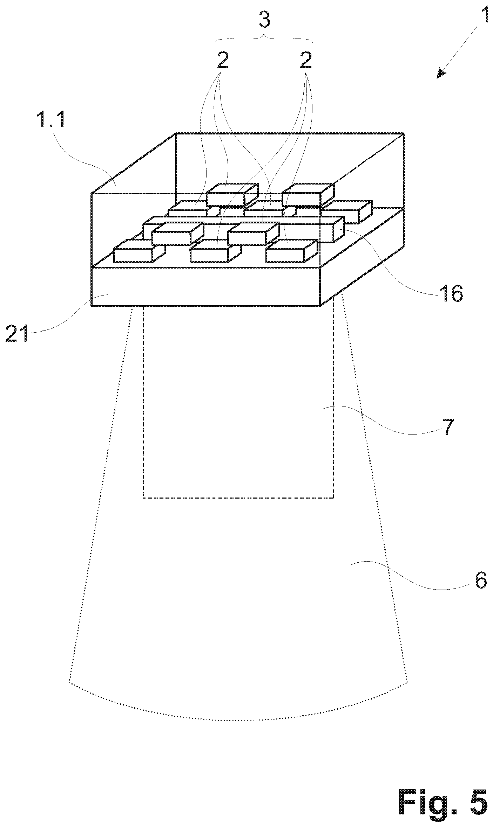

Die Ultraschallsonde 16 kann in weiteren Ausführungen des erfindungsgemäßen Messgeräts 1 auch in dem Array 3 von Strahlungsdetektoren 2 angeordnet sein, wie dies als drittes Ausführungsbeispiel des erfindungsgemäßen Messgeräts 1 in Fig. 5 gezeigt ist. Dargestellt ist der Messkopf 1.1 mit einem Array 3 von Strahlungsdetektoren 2 (nur zehn gezeigt) und einer Ultraschallsonde 16, die zwischen zwei Reihen von Strahlungsdetektoren 2 des Arrays 3 angeordnet ist. An dem Messkopf 1.1 ist zusätzlich ein Kollimator 21 angeordnet, durch dessen Wirkung der Erfassungsbereich 6 des Arrays 3 seitlich eingeschränkt und ein Durchtritt von nicht aus dem Messbereich 7 stammenden Strahlungssignalen 10 (nicht gezeigt, siehe Fig. 1) zu den Strahlungsdetektoren 2 stark reduziert ist.

In weiteren Ausführungen des erfindungsgemäßen Messgeräts 1 können andere Anzahlen von Strahlungsdetektoren 2 in andersgestalteten Arrays 3 angeordnet sein.

### Bezugszeichenliste

- 1: Messgerät
- 1.1: Messkopf
- 1.2: Eintrittsfenster
- 2: Strahlungsdetektor
- 2.1: erster Strahlungsdetektor
- 2.2: zweiter Strahlungsdetektor
- 2.3: dritter Strahlungsdetektor
- 2.4: vierter Strahlungsdetektor
- 2.5: fünfter Strahlungsdetektor
- 3: Array (der Strahlungsdetektoren 2)
- 4: Auswerteeinheit
- 5: Arbeitsstation
- 6: Erfassungsbereich
- 7: Messbereich
- 8: Radionuklid
- 9: Ursprungsort
- 10: Strahlungssignal
- 11: Anzeige
- 12: Vorverstärker
- 13: Modulator
- 14: Diskriminator
- 15: Zählerkarte
- 16: Ultraschallsonde
- 17: Ultraschallgerät
- 18: Ultraschallsignal
- 19: Probekörper
- 20: Bildverarbeitungseinheit
- 21: Kollimator
- D1: erste Detektionskurve
- D2: zweite Detektionskurve
- D3: dritte Detektionskurve
- D4: vierte Detektionskurve
- D5: fünfte Detektionskurve

## Patentansprüche

1. Verfahren zur Bestimmung von Ursprungsorten (9) von Strahlungssignalen (10) in einem Messbereich (7), bei dem
- eine Anzahl n = 2, 3, ..., i von Strahlungsdetektoren (2) zum Empfangen von Strahlungssignalen (10) aus dem Messbereich (7) und zum Bereitstellen von Messwerten aufgrund empfangener Strahlungssignale (10) in einer bekannten relativen räumlichen Anordnung in einem Messkopf (1) zueinander angeordnet werden, indem vordere Strahlungsdetektoren (2.1, 2.3, 2.5) am Eintrittsfenster des Messkopfes stehen und alternierend hierzu, in Lücken zwischen den vorderen Strahlungsdetektoren (2.1, 2.3, 2.5), hintere Strahlungsdetektoren (2.2, 2.4) weiter entfernt vom Eintrittsfenster angeordnet sind, sodass jeder der Strahlungsdetektoren (2) eine voneinander verschiedene geometrische Effizienz hinsichtlich der Erfassung eines Strahlungssignals (10) von einem bestimmten Ursprungsort (9) aufweist,
- mittels der Anzahl von Strahlungsdetektoren (2) zu einem Messzeitpunkt Strahlungssignale (10) aus dem Messbereich (7) erfasst und aufgrund der erfassten Strahlungssignale (10) dem Messzeitpunkt zugeordnete Messwerte bereitgestellt werden,
- die Bestimmung der Ursprungsorte (9) der Strahlungssignale (10) unter Berücksichtigung der Messwerte aller Strahlungsdetektoren (2) und der bekannten relativen Lagebeziehungen der Strahlungsdetektoren (2) zueinander erfolgt und
- die Bestimmung der Ursprungsorte (9) unter Nutzung eines Referenzdatensatzes erfolgt, wobei der Referenzdatensatz aus einer Anzahl von Teilreferenzdatensätzen besteht und jeder Teilreferenzdatensatz durch spezifische Daten jeweils eines der Ursprungsorte (9) gebildet ist und die spezifischen Daten durch Beziehungen von Messwerten der Strahlungsdetektoren (2) zueinander gebildet werden.

2. Verfahren nach Anspruch 1, **dadurch gekennzeichnet, dass** zu einem Messzeitpunkt die Ursprungsorte (9) der erfassten Strahlungssignale (10) bestimmt und für eine graphische Darstellung des Messbereichs (7) mit den Ursprungsorten (9) bereitgestellt werden.

3. Verfahren nach Anspruch 1 oder 2, **dadurch gekennzeichnet, dass**
- zu dem Messzeitpunkt zusätzlich Ultraschallsignale (18) aus dem Messbereich (7) erfasst und als dem Messzeitpunkt zugeordnete Ultraschallmesswerte für eine graphische Darstellung des Messbereichs (7) bereitgestellt werden und
- die Ursprungsorte (9) und die Ultraschallmesswerte zeitgleich, orts- und lagerichtig in einer graphischen Darstellung angezeigt werden.

4. Verfahren nach einem der Ansprüche 1 bis 3, **dadurch gekennzeichnet, dass** die Strahlungsdetektoren (2) ohne einen den Strahlungsdetektoren (2) empfangsseitig vorgeordneten Kollimator (21) angeordnet werden.

5. Messgerät (1) zur simultanen Erfassung von Strahlungsereignissen zerfallender Radionuklide (8) in einem Messbereich (7), bei dem
- in einem Messkopf (1.1) des Messgeräts (1) eine Anzahl n = 2, 3, ..., i von Strahlungsdetektoren (2) in einer bekannten relativen räumlichen Anordnung zueinander, jeweils an einer Messposition angeordnet sind, indem vordere Strahlungsdetektoren (2.1, 2.3, 2.5) am Eintrittsfenster des Messkopfes stehen und alternierend hierzu, in Lücken zwischen den vorderen Strahlungsdetektoren (2.1, 2.3, 2.5), hintere Strahlungsdetektoren (2.2, 2.4) weiter entfernt vom Eintrittsfenster angeordnet sind, sodass jeder der Strahlungsdetektoren (2) zum Empfangen eines Strahlungssignals (10) aus dem Messbereich (7) und zum Bereitstellen eines Messwertens aufgrund des empfangenen Strahlungssignals (10) dient, wobei jeder der Strahlungsdetektoren (2) eine voneinander verschiedene geometrische Effizienz hinsichtlich der Erfassung der Messwerte der Strahlungssignale (10) aufweist, die von unterschiedlichen Ursprungsorten (9) innerhalb des Messbereichs (7) stammen, und
- das Messgerät (1) eine Auswerteeinheit (4) zum Auswerten der Strahlungssignale (10) und zur Bestimmung der Ursprungsorte (9) aufweist, die so konfiguriert ist, dass aus einer Anzahl der Messwerte, die jeweils an nur einer der Messpositionen des Messkopfes (1.1) erfasst wurden und die mit Daten eines Referenzdatensatzes verglichen werden, die Ursprungsorte (9) der erfassten Strahlungssignale (10) ermittelbar sind, wobei der Referenzdatensatz aus einer Anzahl von Teilreferenzdatensätzen besteht und jeder Teilreferenzdatensatz durch spezifische Daten jeweils eines der Ursprungsorte (9) gebildet ist und die spezifischen Daten jedes Teilreferenzdatensatzes durch Beziehungen der Messwerte der Strahlungsdetektoren (2) zueinander gebildet sind.

6. Messgerät (1) nach Anspruch 5,
**dadurch gekennzeichnet,**
**dass** in dem Messkopf (1.1) zusätzlich mindestens eine Ultraschallsonde (16) zum Senden und Empfangen von Ultraschallsignalen (18) aus dem Messbereich (7) angeordnet ist.

7. Messgerät (1) nach einem der Ansprüche 5 oder 6,
**dadurch gekennzeichnet, dass**
das Messgerät (1) als ein kollimatorloses Messgerät (1) ausgebildet ist.

8. Messgerät (1) nach einem der Ansprüche 5 bis 7,
**dadurch gekennzeichnet, dass**
der Messkopf (1.1) ein tragbares Gerät ist und durch eine Hand eines Benutzers haltbar und führbar ist.

9. Messgerät (1) nach Anspruch 6,
**dadurch gekennzeichnet, dass** die
mindestens eine Ultraschallsonde (16) in dem Array (3) von Strahlungsdetektoren (2) angeordnet ist.

10. Messgerät (1) nach Anspruch 6
**dadurch gekennzeichnet, dass** die
mindestens eine Ultraschallsonde (16) neben dem Array (3) von Strahlungsdetektoren (2) angeordnet ist.

11. Messgerät (1) nach Anspruch 6,
**dadurch gekennzeichnet, dass** das
Array (3) von Strahlungsdetektoren (2) hinter der mindestens einen Ultraschallsonde (16) angeordnet ist, sodass die Strahlungssignale (10) durch Bereiche der Ultraschallsonde (16) hindurchtreten, bevor sie durch die Strahlungsdetektoren (2) erfassbar sind.

12. Messgerät (1) nach Anspruch 5, **dadurch gekennzeichnet, dass** die Strahlungsdetektoren (2.1, 2.2, 2.3, 2.4, 2.5) CdZnTe-Halbleiterdetektoren sind und eine Kantenlänge von 4,8 mm aufweisen.

## Claims

1. A method for determining the points of origin (9) of radiation signals (10) in a measurement zone (7), wherein
- a number n = 2, 3, ..., i of radiation detectors (2) for receiving radiation signals (10) from the measurement zone (7) and for providing measured values on the basis of received radiation signals (10) are arranged in a known relative spatial arrangement to one another in a measuring head (1), in that front radiation detectors (2.1, 2.3, 2.5) are located at the entrance window of the measuring head and, alternatingly thereto, in gaps between the front radiation detectors (2.1, 2.3, 2.5), rear radiation detectors (2.2, 2.4) are arranged further away from the entrance window, so that each of the radiation detectors (2) has a mutually different geometric efficiency with respect to the detection of a radiation signal (10) from a specific point of origin (9),
- radiation signals (10) from the measurement zone (7) are detected by means of the number of radiation detectors (2) at a measurement time and measured values assigned to the measurement time are provided on the basis of the detected radiation signals (10),
- the determination of the points of origin (9) of the radiation signals (10) is carried out taking into account the measured values of all radiation detectors (2) and the known relative positional relationships of the radiation detectors (2) to one another, and
- the determination of the points of origin (9) is carried out using a reference data set, said reference data set consisting of a number of partial reference data sets and each partial reference data set being formed by specific data of one of the points of origin (9) in each case and the specific data being formed by relationships of measured values of the radiation detectors (2) to one another.

2. The method according to claim 1, **characterised in that** the points of origin (9) of the detected radiation signals (10) are determined at a measurement time and are provided for a graphical representation of the measurement zone (7) with the points of origin (9).

3. The method according to claim 1 or 2, **characterised in that**
- ultrasonic signals (18) from the measurement zone (7) are additionally detected at the measurement time and are provided as ultrasonic measured values assigned to the measurement time for a graphical representation of the measurement zone (7), and
- the points of origin (9) and the ultrasonic measured values are displayed simultaneously, in the correct location and position in a graphical representation.

4. The method according to any one of claims 1 to 3, **characterised in that** the radiation detectors (2) are arranged without placing a collimator (21) upstream of the radiation detectors (2) on the receiving side.

5. A measuring device (1) for simultaneously detecting radiation events of decaying radionuclides (8) in a measurement zone (7), wherein
- a number n = 2, 3, ..., i of radiation detectors (2) are arranged in a known relative spatial arrangement to one another in a measuring head (1.1), each being arranged in a respective measurement position, in that front radiation detectors (2.1, 2.3, 2.5) are located at the entrance window of the measuring head and, alternatingly thereto, in gaps between the front radiation detectors (2.1, 2.3, 2.5), rear radiation detectors (2.2, 2.4) are arranged further away from the entrance window, so that each of the radiation detectors (2) serves to receive a radiation signal (10) from the measurement zone (7) and to provide a measured value on the basis of the received radiation signal (10), each of the radiation detectors (2) having a mutually different geometric efficiency with respect to the detection of the measured values of the radiation signals (10) originating from different points of origin (9) within the measurement zone (7), and
- the measuring device (1) has an evaluation unit (4) for evaluating the radiation signals (10) and for determining the points of origin (9), which evaluation unit (4) is configured in such a way that the points of origin (9) of the detected radiation signals (10) can be determined from a number of the measured values which have each been detected at only one of the measuring positions of the measuring head (1.1) and which are compared with data of a reference data set, the reference data set comprising a number of partial reference data sets, and
each partial reference data set is formed by specific data of a respective one of the points of origin (9) and the specific data of each partial reference data set are formed by relationships of the measured values of the radiation detectors (2) to one another.

6. The measuring device (1) according to claim 5, **characterised in that** at least one ultrasonic probe (16) for transmitting and receiving ultrasonic signals (18) from the measurement zone (7) is additionally arranged in the measuring head (1.1).

7. The measuring device (1) according to any one of claims 5 or 6, **characterised in that** the measuring device (1) is designed as a collimator-less measuring device (1).

8. The measuring device (1) according to any one of claims 5 to 7, **characterised in that** the measuring head (1.1) is a portable device and is holdable and guidable by a user's hand.

9. The measuring device (1) according to claim 6, **characterised in that** the at least one ultrasonic probe (16) is arranged in the array (3) of radiation detectors (2).

10. The measuring device (1) according to claim 6, **characterised in that** the at least one ultrasonic probe (16) is arranged next to the array (3) of radiation detectors (2).

11. The measuring device (1) according to claim 6, **characterised in that** the array (3) of radiation detectors (2) is arranged behind the at least one ultrasonic probe (16) so that the radiation signals (10) pass through regions of the ultrasonic probe (16) before being detectable by the radiation detectors (2).

12. The measuring device (1) according to claim 5, **characterised in that** the radiation detectors (2.1, 2.2, 2.3, 2.4, 2.5) are CdZnTe semiconductor detectors and have an edge length of 4.8 mm.

## Revendications

1. Procédé pour déterminer des lieux d'origine (9) de signaux de rayonnement (10) dans une zone à mesurer (7), dans lequel
- un nombre n = 2, 3, ..., i de détecteurs de rayonnement (2) pour recevoir des signaux de rayonnement (10) de la zone à mesurer (7) et pour fournir des valeurs de mesure sur la base des signaux de rayonnement reçus (10) sont disposés les uns par rapport aux autres dans une disposition spatiale relative connue dans une tête de mesure (1), en ce que des détecteurs de rayonnement avant (2.1, 2.3, 2. 5) se trouvent à la fenêtre d'entrée de la tête de mesure et, en alternance, dans des espaces entre les détecteurs de rayonnement avant (2.1, 2.3, 2.5), des détecteurs de rayonnement arrière (2.2, 2.4) sont disposés plus loin de la fenêtre d'entrée, de sorte que chacun des détecteurs de rayonnement (2) présente une efficacité géométrique différente les uns des autres en ce qui concerne la détection d'un signal de rayonnement (10) provenant d'un lieu d'origine déterminé (9),
- au moyen du nombre de détecteurs de rayonnement (2), on détecte à un moment de mesure des signaux de rayonnement (10) provenant de la zone à mesurer (7) et, sur la base des signaux de rayonnement détectés (10), on met à disposition des valeurs de mesure associées au moment de mesure,
- la détermination des lieux d'origine (9) des signaux de rayonnement (10) s'effectue en tenant compte des valeurs de mesure de tous les détecteurs de rayonnement (2) et des relations de position relatives connues des détecteurs de rayonnement (2) les uns par rapport aux autres et
- la détermination des lieux d'origine (9) s'effectue en utilisant un ensemble de données de référence, l'ensemble de données de référence étant constitué d'un nombre d'ensembles de données de référence partiels et chaque ensemble de données de référence partiel étant formé par des données spécifiques respectivement d'un des lieux d'origine (9) et les données spécifiques étant formées par des relations de valeurs de mesure des détecteurs de rayonnement (2) entre elles.

2. Procédé selon la revendication 1, **caractérisé en ce qu'**à un moment de mesure, les lieux d'origine (9) des signaux de rayonnement (10) détectés sont déterminés et mis à disposition pour une représentation graphique de la zone à mesurer (7) avec les lieux d'origine (9).

3. Procédé selon la revendication 1 ou 2, **caractérisé en ce que**
- au moment de mesure, des signaux ultrasonores (18) sont en outre détectés à partir de la zone à mesurer (7) et sont mis à disposition en tant que valeurs de mesure ultrasonores associées au moment de mesure pour une représentation graphique de la zone à mesurer (7) et
- les lieux d'origine (9) et les valeurs de mesure ultrasonores sont affichés simultanément, en lieu et position correctes, dans une représentation graphique.

4. Procédé selon l'une quelconque des revendications 1 à 3, **caractérisé en ce que** les détecteurs de rayonnement (2) sont disposés sans collimateur (21) placé en amont des détecteurs de rayonnement (2) du côté de la réception.

5. Appareil de mesure (1) permettant de détecter simultanément des événements radiatifs de radionucléides (8) qui se désintègrent dans une zone à mesurer (7), dans lequel
- dans une tête de mesure (1.1) de l'appareil de mesure (1), un nombre n = 2, 3, ..., i de détecteurs de rayonnement (2) sont disposés dans une disposition spatiale relative connue les uns par rapport aux autres, chacun à une position de mesure, en ce que des détecteurs de rayonnement avant (2.1, 2.3, 2.5) se trouvent à la fenêtre d'entrée de la tête de mesure et, en alternance avec cela, dans des espaces entre les détecteurs de rayonnement avant (2.1, 2.3, 2.5), des détecteurs de rayonnement arrière (2.2, 2.4) sont disposés plus loin de la fenêtre d'entrée, de sorte que chacun des détecteurs de rayonnement (2) sert à recevoir un signal de rayonnement (10) de la zone à mesurer (7) et à fournir une valeur de mesure sur la base du signal de rayonnement reçu (10), chacun des détecteurs de rayonnement (2) ayant une efficacité géométrique différente les uns des autres en ce qui concerne la détection des valeurs de mesure des signaux de rayonnement (10) provenant de différentes lieux d'origine (9) dans la zone à mesurer (7), et
- l'appareil de mesure (1) présente une unité d'évaluation (4) pour évaluer les signaux de rayonnement (10) et pour déterminer les lieux d'origine (9), qui est configurée de telle sorte que les lieux d'origine (9) des signaux de rayonnement (10) détectés peuvent être déterminés à partir d'un nombre de valeurs de mesure qui ont été détectées respectivement à seulement l'une des positions de mesure de la tête de mesure (1.1) et qui sont comparées à des données d'un ensemble de données de référence, l'ensemble de données de référence étant constitué d'un nombre d'ensembles de données de référence partiels et
chaque ensemble de données de référence partiel est formé par des données spécifiques respectivement de l'un des lieux d'origine (9) et les données spécifiques de chaque ensemble de données de référence partiel sont formées par des relations entre les valeurs de mesure des détecteurs de rayonnement (2).

6. Appareil de mesure (1) selon la revendication 5, **caractérisé en ce que** dans la tête de mesure (1.1) est disposée en plus au moins une sonde à ultrasons (16) pour l'émission et la réception de signaux ultrasonores (18) provenant de la zone à mesurer (7).

7. Appareil de mesure (1) selon l'une quelconque des revendications 5 ou 6, **caractérisé en ce que** l'appareil de mesure (1) est conçu comme un appareil de mesure (1) sans collimateur.

8. Appareil de mesure (1) selon l'une quelconque des revendications 5 à 7, **caractérisé en ce que** la tête de mesure (1.1) est un appareil portable et peut être maintenue et guidée par une main d'un utilisateur.

9. Appareil de mesure (1) selon la revendication 6, **caractérisé en ce que** ladite au moins une sonde à ultrasons (16) est disposée dans le réseau (3) de détecteurs de rayonnement (2).

10. Appareil de mesure (1) selon la revendication 6, **caractérisé en ce que** ladite au moins une sonde à ultrasons (16) est disposée à côté du réseau (3) de détecteurs de rayonnement (2).

11. Appareil de mesure (1) selon la revendication 6, **caractérisé en ce que** le réseau (3) de détecteurs de rayonnement (2) est disposé derrière ladite au moins une sonde à ultrasons (16), de sorte que les signaux de rayonnement (10) traversent des régions de la sonde à ultrasons (16) avant de pouvoir être détectés par les détecteurs de rayonnement (2).

12. Appareil de mesure (1) selon la revendication 5, **caractérisé en ce que** les détecteurs de rayonnement (2.1, 2.2, 2.3, 2.4, 2.5) sont des détecteurs à semiconducteur CdZnTe et présentent une longueur d'arête de 4,8 mm.
